# EUROPÄISCHE PATENTANMELDUNG

(11) **EP 2 404 547 A1**
(43) Veröffentlichungstag der Anmeldung: **11.01.2012**
(21) Anmeldenummer: 10007139.8
(22) Anmeldetag: 10.07.2010
(51) Int. Cl.: A61B 5/00, A61B 17/34, A61M 5/158, A61M 5/42

(54) **Verfahren zum Vorbereiten eines Einstichs einer Insertionsnadel in Unterhautfettgewebe und Insertionsvorrichtung hierfür**

(71) Anmelder: Roche Diagnostics GmbH, 68305 Mannheim (DE); F. Hoffmann-La Roche AG, 4070 Basel (CH)
(72) Erfinder: Deck, Frank, 67150 Niederkirchen (DE); Ebert, Karl-Peter, 64407 Fraenkisch-Crumbach (DE)
(74) Vertreter: Twelmeier Mommer & Partner

(57) **Zusammenfassung**

Die Erfindung betrifft ein Verfahren zum Vorbereiten eines Einstichs einer Insertionsnadel (8) in Unterhautfettgewebe eines Patienten, wobei eine Insertionsvorrichtung (3), welche die Insertionsnadel (8) enthält, an die Haut eines Patienten angelegt wird. Erfindungsgemäß ist vorgesehen, dass die Haut durch Einwirkung der Insertionsvorrichtung (3) verformt und dabei eine Hautfläche (1a) stufen- oder hangartig ansteigend so angeordnet wird, dass die Insertionsnadel (8) auf die stufen- oder hangartig ansteigende Hautfläche (1a) deutet. Die Erfindung betrifft ferner eine Insertionsvorrichtung (3) zur Durchführung dieses Verfahrens.

## Beschreibung

Die Erfindung betrifft ein Verfahren zum Vorbereiten eines Einstichs einer Insertionsnadel in Unterhautfettgewebe eines Patienten mittels einer Insertionsvorrichtung sowie eine Insertionsvorrichtung.

Sensoren zur Messung von Analytkonzentrationen in-vivo, beispielsweise der Glucosekonzentration, werden in unter der Haut liegendes Fettgewebe eines Patienten insertiert, indem eine Nadel in das Fettgewebe eingestochen wird. Hierfür gebräuchliche Insertionsnadeln sind in der Regel als Hohlnadeln oder V-förmige Rinnen ausgebildet, in denen ein Sensor liegt, beispielsweise ein Elektrodensystem für elektrochemische Messungen. Nach dem Einstich wird die Insertionsnadel aus dem Körpergewebe herausgezogen, wobei der Sensor in der erzeugten Stichwunde verbleibt.

Insertionsvorrichtungen bestehen häufig aus einer Basiseinheit, die auf den Körper eines Patienten aufgesetzt wird, und einem Stechgerät, das für eine Insertion an die Basiseinheit angekoppelt und anschließend wieder abgenommen wird. Daneben sind auch Insertionsvorrichtungen bekannt, die nur aus einem Stechgerät bestehen.

Insertionssysteme werden häufig von Patienten selbst bedient, beispielsweise um Sensoren zur Messung der Glucosekonzentration zu insertieren. Bei der Entwicklung von Insertionssystemen ist es deshalb ein ständiges Ziel, dass diese möglichst einfach und sicher bedient werden können, Sensoren zuverlässig in Fettgewebe verankern und präzise Messungen ermöglichen. Zudem soll der mit dem Einstich einer Insertionsnadel verbundene Schmerz möglichst weitgehend minimiert werden.

Diese Aufgabe wir durch ein Verfahren mit den im Anspruch 1 angegebenen Merkmalen sowie durch eine Insertionsvorrichtung gemäß Anspruch 4 gelöst. Vorteilhafte Weiterbildungen der Erfindung sind Gegenstand von Unteransprüchen.

Während herkömmliche Insertionsvorrichtungen einfach auf die Haut eines Patienten aufgesetzt werden und anschließend eine Insertionsnadel schräg oder senkrecht zur Hautoberfläche in Unterhautfettgewebe einstechen, wird erfindungsgemäß vor dem Auslösen eines Stichs die Einstichstelle vorbereitet, indem die Haut verformt und dabei eine Hautfläche stufen- oder hangartig ansteigende aufgerichtet wird. Auf diese Weise kann eine Insertionsnadel bei einem Stich unter einem steilen Winkel, idealer Weise im wesentlichen senkrecht, auf die stufen- oder hangartig ansteigende Hautfläche auftreffen und dennoch auf einer verhältnismäßig großen Länge in das Fettgewebe eindringen, bevor die Gefahr einer Verletzung von Muskelgewebe besteht. Erfindungsgemäß werden also die Hautoberfläche und darunter liegendes Fettgewebe zur Vorbereitung einer Insertion geformt, um vorteilhafte Bedingungen für einen Einstich zu erzeugen.

Beispielsweise kann zum Vorbereiten eines Einstichs einer Insertionsnadel in Unterhautfettgewebe eine Unterseite der Insertionsvorrichtung gegen die Haut gedrückt werden, wobei die Unterseite zwei Andruckbereiche aufweist, zwischen denen eine Aussparung zum Austreten der Insertionsnadel ist und die auf unterschiedlichen Höhen angeordnet sind. Dies bedeutet, dass eine von oben nach unten verlaufende Schnittebene, in welcher die Nadel liegt, den ersten Andruckbereich in einer ersten Linie und den zweiten Andruckbereich in einer zweiten Linie schneidet, und die erste Linie in der Nachbarschaft der Aussparung in einer größeren Höhe als die zweite Linie verläuft. Die Haut und darunterliegendes Fettgewebe werden deshalb beim Anpressen der Insertionsvorrichtung an einen Patienten in der Nachbarschaft der Aussparung in dem zweiten Andruckbereich stärker komprimiert als in dem ersten Andruckbereich. Im Bereich der Aussparung bildet sich deshalb eine stufen- oder hangartig ansteigende Hautfläche, auf welche die in der Insertionsvorrichtung enthaltene Insertionsnadel deutet.

Die beiden Andruckbereiche können eben ausgebildet sein. Dabei kann an der Unterseite der Insertionsvorrichtung zwischen den beiden Andruckbereichen eine beispielsweise stufen- oder rampenartig ansteigende Fläche vorgesehen sein, in der die Aussparung für die Insertionsnadel angeordnet ist. Möglich ist es auch, dass die beiden Andruckbereiche gekrümmte Flächen sind, die bei der Aussparung aneinander angrenzen.

Ein Komprimieren von Gewebe ist vorteilhaft, da sich die Haut dann leichter durchstechen lässt, ist aber nicht unbedingt erforderlich. Beispielsweise kann die Haut aufgewölbt werden, indem eine Klebefläche auf die zu verformende Haut aufgeklebt und anschließend nach oben gezogen wird. Ein Aufwölben der Haut kann aber auch durch Ausüben von Druck bewirkt oder unterstützt werden, so dass dabei Gewebe komprimiert wird. Eine Flanke einer Aufwölbung der Haut bildet eine hangartig ansteigende Fläche, die eine vorteilhafte Insertion ermöglicht. Die Mittel zum Formen einer stufen- oder hangartig ansteigenden Hautfläche können also beispielsweise als Mittel zum Erzeugen einer Hautwölbung ausgebildet sein.

Eine Insertionsnadel kann die Haut im Allgemeinen umso leichter durchstoßen, je steiler der Winkel ist, mit dem sie auf die Haut trifft. Am Ende eines erfindungsgemäßen Verfahrens schließt eine Flächennormale der Hautoberfläche in dem Punkt, auf den die Insertionsnadel mit ihrer Spitze deutet, mit der Insertionsnadel deshalb bevorzugt einen Winkel von weniger als 30°, besonders bevorzugt weniger als 20°, insbesondere weniger 10°, ein.

Bevorzugt wird die Hautoberfläche bei einem erfindungsgemäßen Verfahren durch Einwirkung der Insertionsvorrichtung mindestens so stark verformt, dass eine Flächennormale in einem Punkt der ansteigenden Fläche, auf den die Insertionsnadel deutet, mit einer Flächennormalen in demselben Punkt der Hautfläche vor Verformung einen Winkel von wenigstens 45°, vorzugsweise wenigstens 60°, einschließt.

Bevorzugt ist die Insertionsnadel in der Insertionsvorrichtung schräg von oben nach unten gehalten, erstreckt sich also schräg zur Unterseite der Insertionsvorrichtung. Die Worte "unten" und "oben" beziehen sich dabei auf die Insertionsvorrichtung, wobei die Unterseite der Insertionsvorrichtung jene Seite ist, die bei einem Stich bzw. nach Abschluss des erfindungsgemäßen Verfahrens auf der Haut des Patienten sitzt. Die Oberseite der Insertionsvorrichtung ist dementsprechend die bei einem Stich bzw. nach Abschluss des erfindungsgemäßen Verfahrens von der Haut des Patienten abgewandte Seite.

Eine erfindungsgemäße Insertionsvorrichtung hat Mittel zum Formen einer stufen- oder hangartig ansteigen Hautfläche, die der für den Austritt der Insertionsnadel vorgesehenen Aussparung zugewandt ist. Die Insertionsvorrichtung formt die Haut also so, dass eine Hautfläche stufen- oder hangartig aufgerichtet wird und dann der Aussparung, beispielsweise eine Austrittsöffnung zugewandt ist. Bevorzugt wird die Haut von einer erfindungsgemäßen Insertionsvorrichtung so geformt, dass die stufen- oder hangartig ansteigende Hautfläche an der Aussparung anliegt, da auf diese Weise die Einstichstelle besonders gut definiert ist.

Die Mittel zum Formen einer stufen- oder hangartig ansteigen Hautfläche können beispielsweise Mittel zum Aufrechterhalten einer Aufwölbung der Haut des Patienten sein. Eine in der Insertionsvorrichtung enthaltende Insertionsnadel ist dabei bevorzugt schräg zu einer Auflage- oder Andruckfläche der Insertionsvorrichtung orientiert, neben der die Aufwölbung erzeugt wird. Dies bedeutet, dass die Insertionsnadel in eine Richtung deutet, welche mit einer plan ausgebildeten Auflage- oder Andruckfläche einen spitzen Winkel, beispielsweise einen Winkel zwischen 20° und 60° einschließt. Da Insertionsnadeln üblicher Weise einen geradlinigen Verlauf haben, schließt dann auch die Insertionsnadel mit der Auflagefläche einen spitzen Winkel ein. An sich kann die Auflage- oder Andruckfläche auch etwas gerundet sein, beispielsweise an die natürliche Rundung eines Bauchs angepasst sein. In einem solchen Fall, bedeutet die schräge Orientierung der Insertionsnadel, dass eine tangentiale Verlängerung der Fläche die Richtung, in welche die Insertionsnadel deutet, unter einem spitzen Winkel schneidet.

Als Mittel zum Formen der Haut können beispielsweise zwei gegeneinander bewegliche Halteteile verwendet werden, zwischen denen einen Hautwölbung gefasst wird. Die Mittel können aber beispielsweise auch ein haubenartiges Gehäuse sein, das einen Unterdruckanschluss aufweist, so dass durch Ansaugen eine Hautwölbung erzeugt und erhalten werden kann.

Werden zwei gegeneinander bewegliche Halteteile verwendet, um eine Hautwölbung zu erzeugen und zu halten, weisen diese bevorzugt jeweils einen Andruckbereich für die Hautwölbung auf. Bevorzugt deutet die Insertionsnadel in einem Abstand an wenigstens einem der Andruckbereiche entlang. Bei einem Stich schiebt die Insertionsvorrichtung die Insertionsnadel dann in einem Abstand von dem Andruckbereich an dieser entlang, bevorzugt im Wesentlichen parallel zu dem Andruckbereich. Die Insertionsnadel kann so in Unterhautfettgewebe parallel zur Hautoberfläche eingeschoben werden.

Möglich ist dabei, dass der Andruckbereich des anderen Halteteils eine Aussparung, insbesondere eine Öffnung für die Insertionsnadel aufweist. Möglich ist es aber auch, dass die Insertionsnadel zwischen den beiden Andruckbereichen hindurch deutet, also die Insertionsnadel bei einem Stich in einem Abstand an beiden Andruckbereichen vorbei geführt wird.

Eine vorteilhafte Weiterbildung der Erfindung sieht vor, dass die Insertionsvorrichtung aus einem ersten und einem zweiten Teilkörper zusammengesetzt ist, wobei der erste und der zweite Teilkörper gegeneinander beweglich sind und eine Fuge zwischen dem ersten und dem zweiten Teilkörper durch die für den Austritt der Insertionsnadel vorgesehene Aussparung hindurch verlauft. Indem die beiden Teilkörper gegeneinander beweglich sind, kann nach einem Stich die durch die Aussparung verlaufende Fuge vergrößert werden, damit sich die Insertionsvorrichtung leichter von dem Patienten abnehmen lässt, beispielsweise um das Handhaben eines mit dem Sensor verbundenen Sensorpatches zu erleichtern. Sensoren sind häufig mit einem sogenannten Sensorpatch verbunden, das nach der Insertion eines Sensors auf die Haut des Patienten aufgeklebt wird. Ein Sensorpatch kann beispielsweise einen Potentiostaten zur Energieversorgung eines den Sensor bildenden Elektrodensystems, eine Batterie und/oder eine Steuerungselektronik enthalten.

Bevorzugt können die beiden Teilkörper von einander abgenommen werden. Die zwischen ihnen verlaufende Fuge kann aber beispielsweise auch durch eine Schwenkbewegung der beiden Teilkörper zueinander soweit verbreitert werden, dass sich ein mit dem insertierten Sensor verbundenes Sensorpatch auf der Haut des Patienten platzieren lässt.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen versehen. Es zeigen:
- Fig. 1: eine schematische Darstellung einer aufgewölbten Hautoberfläche mit einem insertierten Sensor;
- Fig. 2: die in Figur 1 dargestellte Hautoberfläche im entspannten Zustand mit insertiertem Sensor;
- Fig. 3: eine schematische Darstellung eines Ausführungsbeispiels einer Insertionsvorrichtung in einem zum Aufsetzen auf die Haut eines Patienten vorgesehenen Ausgangszustand;
- Fig. 4: die in Figur 3 gezeigte Insertionsvorrichtung in einem Endzustand zum Halten einer Hautwölbung;
- Fig. 5: die in Figur 4 dargestellte Insertionsvorrichtung mit ausgefahrener Insertionsnadel;
- Fig. 6: eine schematische Darstellung eines weiteren Ausführungsbeispiels einer Insertionsvorrichtung in einem Ausgangszustand;
- Fig. 7: die in Figur 6 dargestellte Insertionsvorrichtung in einem Endzustand mit ausgefahrener Insertionsnadel;
- Fig. 8: eine schematische Darstellung eines weiteren Ausführungsbeispiels einer Insertionsvorrichtung in einem Ausgangszustand;
- Fig. 9: die in Figur 8 dargestellte Insertionsvorrichtung in einem zum Halten einer Hautaufwölbung vorgesehenen Endzustand;
- Fig. 10: die in Figur 9 gezeigte Vorrichtung mit ausgefahrener Insertionsnadel;
- Fig. 11: eine schematische Darstellung eines weiteren Ausführungsbeispiels einer Insertionsvorrichtung mit einer Hautoberfläche;
- Fig. 12: die in Figur 11 dargestellte Insertionsvorrichtung mit einer Aufwölbung der Haut in einer schematischen Schnittansicht;
- Fig. 13: eine schematische Darstellung eines weiteren Ausführungsbeispiels einer Insertionsvorrichtung in einem Ausgangszustand;
- Fig. 14: die in Figur 13 gezeigte Insertionsvorrichtung mit einer erzeugten Hautaufwölbung;
- Fig. 15: eine schematische Darstellung eines weiteren Ausführungsbeispiels einer Insertionsvorrichtung in einem Ausgangszustand;
- Fig. 16: die in Figur 15 dargestellte Insertionsvorrichtung in einem Endzustand;
- Fig. 17: ein weiteres Ausführungsbeispiel einer Insertionsvorrichtung;
- Fig. 18: ein weiteres Ausführungsbeispiel einer Insertionsvorrichtung;
- Fig. 19: ein weiteres Ausführungsbeispiel einer Insertionsvorrichtung in seinem Ausgangszustand;
- Fig. 20: die in Figur 19 gezeigte Insertionsvorrichtung in einem Endzustand;
- Fig. 21: die in Figur 20 gezeigte Insertionsvorrichtung nach Entfernen des ersten Teilkörpers;
- Fig. 22: die in Figur 21 gezeigte Insertionsvorrichtung nach Entfernen einer seitlichen Stützfläche; und
- Fig.23: einen mit der in Figur 22 dargestellten Insertionsvorrichtung insertierten Sensor und dazugehörendes Sensorpatch.

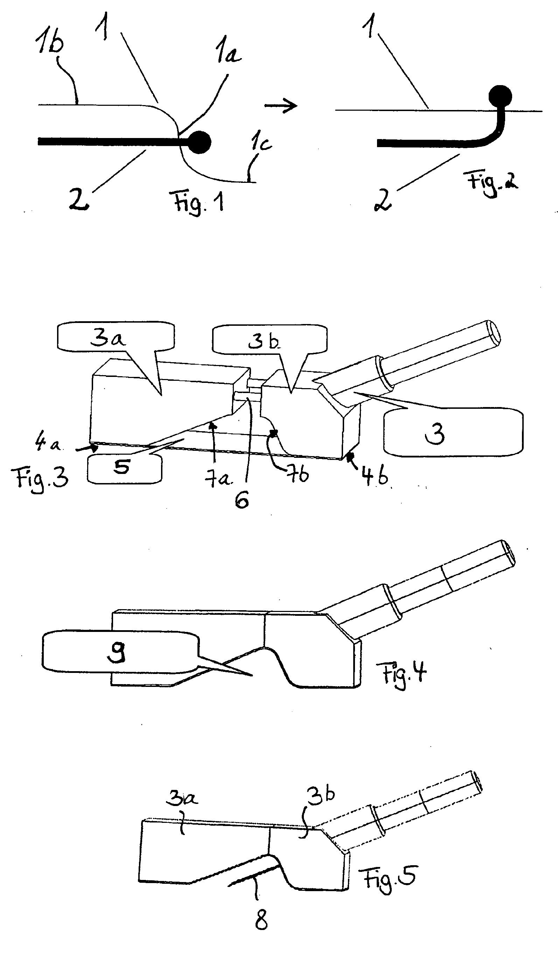

Figur 1 zeigt eine Hautoberfläche 1, in der durch Verformung eine stufen- oder hangartig ansteigende Hautfläche 1 a erzeugt wurde mit einem insertierten Sensor 2. Der Sensor 2 ragt aus der stufen- oder hangartig ansteigenden Hautfläche 1 a heraus und erstreckt sich im Unterhautfettgewebe im Wesentlichen parallel zu einer darüberliegenden Hautfläche 1b, die oben an die oben stufen- oder hangartig ansteigende Hautfläche 1a anschließt.

Ein stufen- oder hangartiger Anstieg bleibt nur bestehen, solange eine entsprechende Kraft auf die Hautoberfläche 1 wirkt und die Hautoberfläche 1 mit darunter liegendem Fettgewebe entsprechend verformt. Wenn eine solche Kraft nicht mehr wirkt, beispielsweise weil eine die Verformung bewirkende Insertionsvorrichtung von der Hautoberfläche 1 abgenommen wurde, entspannt sich die Hautoberfläche 1 und kehrt in ihren natürlichen, nährungsweise ebenen Zustand, zurück. Ein in das Unterhautfettgewebe insertierter Sensor wird dabei verbogen, wie dies in Figur 2 dargestellt ist. Der sich zuvor parallel zu der Hautfläche 1b erstreckende Abschnitt des Sensors ist auch bei entspannter Hautoberfläche 1 nährungsweise parallel zu der Hautoberfläche 1 orientiert und kann sich deshalb auf einer vorteilhaft großen Länge in Fettgewebe unter der Haut erstrecken.

Zum Vorbereiten eines Einstichs einer Insertionsnadel in Unterhautfettgewebe eines Patienten mittels einer Insertionsvorrichtung wird also die Haut durch Einwirkung der Insertionsvorrichtung verformt und dabei eine Hautfläche stufen- oder hangartig ansteigend so angeordnet, dass eine in der Insertionsvorrichtung gehaltene Insertionsnadel auf die stufen- oder hangartig ansteigende Hautfläche 1 a deutet.

Eine Flächennormale der Hautfläche 1 a in dem Punkt, auf den die Insertionsnadel deutet, schließt dabei mit einer gedachten Verlängerung der Insertionsnadel einen Winkel von beispielsweise weniger als 30°, idealer Weise einen Winkel von 0°, ein. Die Insertionsnadel ist also schräg zu der Hautoberfläche 1b, 1c neben der stufen- oder hangartig ansteigenden Hautfläche 1a orientiert. Dies bedeutet, dass die Insertionsnadel in Bezug auf Flächennormalen der Hautoberfläche 1b, 1c neben der Hautaufwölbung einen größeren Winkel einschließt, beispielsweise einen Winkel zwischen 30° und 60°:
Eine stufen- oder hangartig ansteigende Hautfläche 1 a kann zum Vorbereiten einer Insertion eines Sensors mit einer Insertionsvorrichtung auf unterschiedliche Weise erzeugt werden. Nachstehend werden einige Ausführungsbeispiele von Insertionsvorrichtungen erläutert, mit denen eine stufen- oder hangartig ansteigende Hautfläche 1a erzeugt und für die Dauer eines Insertionsvorgangs aufrecht erhalten werden kann.

Figur 3 zeigt ein Ausführungsbeispiel einer Insertionsvorrichtung 3 im Ausgangszustand, in dem die Vorrichtung 3 auf die Haut eines Patienten aufgesetzt wird. Die Insertionsvorrichtung 3 hat zwei Halteteile 3a, 3b, die gegeneinander beweglich sind, um eine Hautwölbung mit einer hangartig ansteigenden Hautfläche zu erzeugen und anschließend zu halten. Die beiden Halteteile 3a, 3b haben jeweils einen Andruckbereich 4a bzw. 4b, mit dem die Insertionsvorrichtung 3 zur Vorbereitung einer Insertion gegen die Haut eines Patienten gedrückt wird. An den Andruckbereichen 4a, 4b der beiden Teilkörper 3a, 3b ist eine Klebefolie 5 zum Aufkleben auf die zu wölbende Haut befestigt. Die Klebefolie 5 ist an den beiden Halteteilen 3a, 3b befestigt. Die Klebefolie 5 überbrückt also eine Fuge zwischen den beiden Halteteilen 3a, 3b. Bei dem dargestellten Ausführungsbeispiel sind die Halteteile 3a, 3b durch Verschiebung gegeneinander beweglich und hierzu über eine Linearführung 6, beispielsweise einer oder mehreren Führungsstangen, miteinander verbunden.

Zum Erzeugen einer Hautwölbung werden die beiden Halteteile 3a, 3b in den in Figur 4 und 5 gezeigten Endzustand zusammengeschoben. Dabei wölbt sich die Klebefolie 5 und damit auch die mit ihr verklebte Haut nach oben, so dass die Klebefolie 5 an Anlageflächen 7a, 7b der Halteteile 3a, 3b anliegt und eine Hautwölbung 9 von den Teilkörpern 3a, 3b gefasst wird. Die Flanken der Hautwölbung 9 sind hangartig ansteigende Hautflächen.

Bei dem dargestellten Ausführungsbeispiel trägt das Halteteil 3b die Insertionsnadel 8. Ein die Insertionsnadel 8 enthaltender Teil der Insertionsvorrichtung kann fest mit dem ersten Halteteil 3a verbunden sein oder erst für den eigentlichen Insertionsvorgang, d.h. den Stich, an dem ersten Halteteil 3a angebracht werden.

Bei dem dargestellten Ausführungsbeispiel hat die Anlagefläche 7b des Halteteils 3b eine Aussparung, nämlich eine Öffnung für die in Figur 5 dargestellte Insertionsnadel 8. Die Insertionsnadel 8 trifft bei einem Stich auf eine seitliche Fläche der Hautwölbung 9 auf, bevorzugt nahezu senkrecht, und bewegt sich dann unter einem flachen Winkel, bevorzugt parallel, zu einer gegenüberliegenden Seitenfläche der Hautwölbung 9, die an der Anlagefläche 7a des Halteteils 3a anliegt. Die Insertionsnadel 8 wird bei dem dargestellten Ausführungsbeispiel im Wesentlichen parallel zu der Anlagefläche 7a des Haltetils 3b in Unterhautfettgewebe eingeschoben.

Bevorzugt hat bei einer Insertionsvorrichtung 3, die wie das dargestellte Ausführungsbeispiel aus zwei gegeneinander verschiebbaren Halteilen 3a, 3b besteht, die Anlagefläche 7b des die Insertionsnadel 8 tragenden Halteteils 3b eine kleinere Länge als die Anlagefläche 7a des anderen Halteteils 3a. Die Länge der Anlageflächen 7a, 7b ist dabei jeweils von dem benachbarten Andruckbereich 4a bzw. 4b in Richtung zu dem anderen Halteteils zu messen. Bevorzugt ist die längere Anlagefläche 7a mindestens doppelt so lang wie die kürzere Anlagefläche 7b.

Die Andruckbereiche 4a, 4b der beiden Halteteile 3a, 3b sind bevorzugt im Wesentlichen eben und parallel zueinander orientiert. Die Anlageflächen 7a, 7b zum Formen und Halten einer Hautwölbung sind bei dem dargestellten Ausführungsbeispiel ebenfalls im Wesentlichen eben, können jedoch auch gekrümmt ausgebildet sein. Die Anlageflächen 7a, 7b steigen ausgehend von den Andruckbereichen 4a, 4b nach oben an, so dass diese eine Ausnehmung an der Unterseite der Insertionsvorrichtung 3 begrenzen, die eine Hautwölbung aufnehmen kann. Die Andruckbereiche 4a, 4b sind jeweils als geradlinige Streifen ausgebildet.

Wenn die Insertionsvorrichtung 3 auf die Hautoberfläche 1 eines Patienten aufgesetzt wird, deutet die Insertionsnadel 8 zunächst schräg auf einen Punkt der noch nicht aufgewölbten Hautoberfläche. Dieses bedeutet, dass eine in Stichrichtung gedachte Verlängerung der Insertionsnadel 8 mit einer Flächennormalen in diesem Punkt der Hautoberfläche 1 einen ersten Winkel von beispielsweise 30° bis 60° einschließt. Nach Erzeugen der Hautaufwölbung deutet die Insertionsnadel 8 auf eine seitliche Flanke der Hautaufwölbung, also auf eine hangartig ansteigende Hautfläche. Eine Flächennormale der Hautfläche in dem Punkt, auf den die Insertionsnadel 8 deutet, schließt dann mit einer gedachten Verlängerung der Insertionsnadel 8 in Stichrichtung einen zweiten Winkel ein, der kleiner als der erste Winkel ist und bevorzugt weniger als 30°; insbesondere weniger als 20° beträgt. Im Idealfall deutet die Insertionsnadel 8 senkrecht auf die hangartig ansteigende Hautfläche.

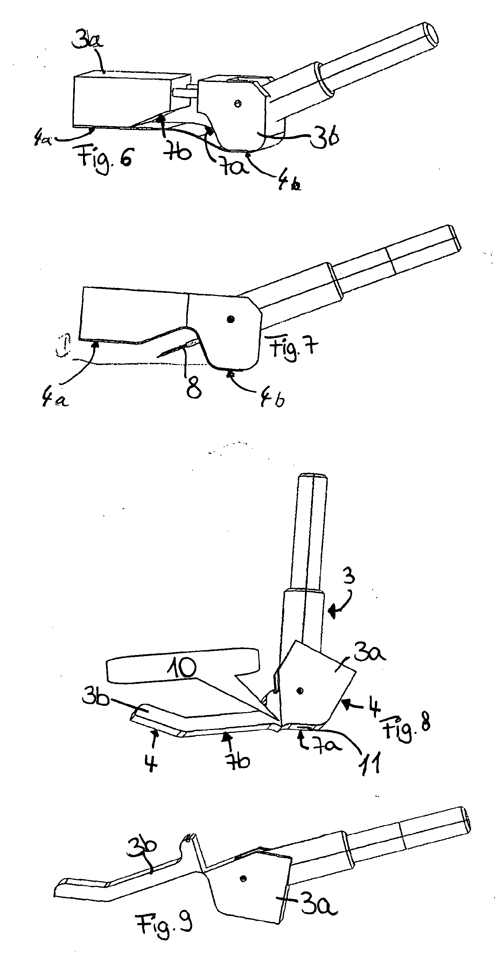

Figur 6 zeigt schematisch ein weiteres Ausführungsbeispiel einer Insertionsvorrichtung 3 im Ausgangszustand, in dem die Insertionsvorrichtung 3 auf den Körper eines Patienten aufgesetzt werden kann. Das in Figur 6 dargestellte Ausführungsbeispiel unterscheidet sich von dem vorstehend beschriebenen Ausführungsbeispiel im Wesentlichen nur dadurch, dass die beiden Andruckbereiche 4a, 4b zueinander versetzt auf unterschiedlichen Höhen angeordnet sind. Eine gedachte Verlängerung des Andruckbereichs 4a des Halteteils 3a schneidet deshalb die Anlagefläche 7b des Halteteil 3b, das die Öffnung 11 für die Insertionsnadel 8 aufweist.

Durch den Versatz der beiden Andruckbereiche 4a, 4b wird vorteilhaft beim Andrücken der Insertionsvorrichtung 3 an die Haut eines Patienten das Gewebe im Bereich der hangartig ansteigenden Hautfläche stärker komprimiert. Dies hat den Vorteil, dass das Gewebe beim Einstechen der Insertionsnadel 8 weniger ausweichen kann und sich ein Sensor 2 präziser im Unterhautfettgewebe platzieren lässt.

Ein weiteres Ausführungsbeispiel einer Insertionsvorrichtung 3 ist in Figur 8 in seiner zum Aufsetzen auf die Haut eines Patienten vorgesehenen Ausgangsstellung und in Figur 9 in seiner zum Halten einer Hautwölbung 9 vorgesehenen Endzustand dargestellt. Dieses Ausführungsbeispiel unterscheidet sich von dem Ausführungsbeispiel der Figuren 3 bis 5 im Wesentlichen dadurch, dass die beiden Halteteile 3a, 3b schwenkbar gegeneinander beweglich sind. Die Insertionsvorrichtung 3 wird zunächst mit den beiden Anlageflächen 7a, 7b auf die Haut eines Patienten aufgesetzt. Die beiden Anlageflächen 7a, 7b sind Klebeflächen, beispielsweise indem die Halteteile 3a, 3b an dieser Stelle mit einer Klebefolie 5 bedeckt sind. Anschließend wird das Halteteil 3a gegenüber dem anderen Halteteil 3b verschwenkt, so dass die Halteteile jeweils mit Ihren Andruckbereichen 4 gegen die Haut des Patienten drücken. Dabei wird die an den Anlageflächen 7a, 7b klebende Haut nach oben gezogen und so eine Hautwölbung 9 zwischen den beiden Halteteilen 3a, 3b erzeugt und gehalten. Die Hautwölbung 9 bildet eine hangartig ansteigende Hautfläche, die an einer in der Anlagefläche 7a vorhanden Öffnung 11 für die Insertionsnadel anliegt

Die beiden Halteteile 3a, 3b können über ein Folienscharnier 10 miteinander verbunden sein, das beispielsweise von der die Anlagenflächen 7a, 7b bedeckenden Klebefolie gebildet werden kann.

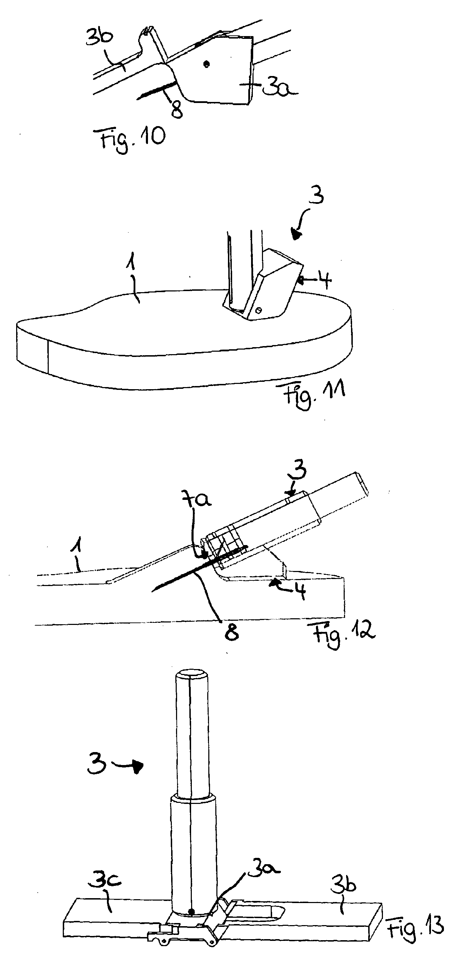

Figur 10 zeigt einen Ausschnitt der in Figur 9 dargestellten Insertionsvorrichtung 3 mit ausgetretener Insertionsnadel 8. Ersichtlich unterscheidet sich zur Orientierung der Insertionsnadel 8 in Bezug auf die Andruckbereiche 4 und die Anlageflächen 7a, 7b nicht von den geometrischen Verhältnissen des in Figur 5 gezeigten Ausführungsbeispiels.

Die Figuren 11 und 12 zeigen ein weiteres Ausführungsbeispiel einer Insertionsvorrichtung 3 mit einer Hautoberfläche 1. Die Insertionsvorrichtung 3 wird zunächst mit der Anlagefläche 7a, die als eine Klebefläche ausgebildet ist und eine Öffnung für die Insertionsnadel aufweist, auf die Haut aufgesetzt. Anschließend wird die Insertionsvorrichtung 3 aus der in Figur 10 gezeigten Ausgangsposition in die in Figur 11 gezeigte Endposition gekippt, so dass eine Andruckfläche 4 der Insertionsvorrichtung 3 gegen die Haut 1 gedrückt wird. Die Andruckfläche 4 schließt bei dem in Figur 11 dargestellten Ausführungsbeispiel mit der Anlagefläche 7a einen stumpfen Winkel ein. Da die Haut 1 an der Anlagefläche 7a klebt, wird durch das Kippen der Insertionsvorrichtung in die in Figur 12 gezeigten Endposition eine Hautwölbung 9 erzeugt, in die anschließend mit einer Insertionsnadel 8 eingestochen werden kann.

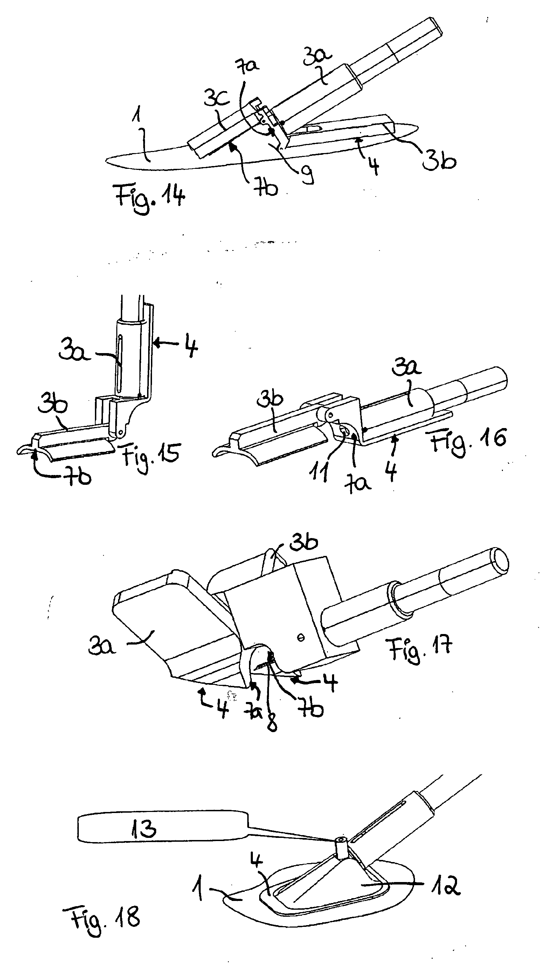

Figur 13 zeigt ein weiteres Ausführungsbeispiel einer Insertionsvorrichtung 3, bei der die Mittel zum Erzeugen und Halten einer Hautwölbung 9 zueinander bewegliche Halteteile 3a, 3b, 3c umfassen. Die Halteteile 3b und 3c sind schwenkbar an dem Halteteil 3a angebracht. Die Insertionsvorrichtung 3 wird in dem in Figur 13 dargestellten Ausgangszustand auf die Haut eines Patienten aufgesetzt. Anschließend wird das Halteteil 3a ähnlich wie bei den vorstehend beschriebenen Ausführungsbeispiel gekippt. Dabei werden die beiden Halteteile 3b und 3c in den in Figur 14 dargestellten Endzustand geschwenkt. Das Halteteil 3b weist die Auflagefläche 4 auf, mit der die Insertionsvorrichtung 3 gegen die Haut gedrückt wird. Das Halteteil 3a bildet eine Anlagefläche 7a für die Hautwölbung 9 und weist eine Öffnung für die Insertionsnadel auf. Das Halteteil 3c bildet eine Anlagefläche 7b, die auf der anderen Seite der Hautwölbung 9 anliegt.

Ein weiteres Ausführungsbeispiel einer Insertionsvorrichtung 3 ist in Figur 15 in seinem Ausgangszustand, in dem es auf die Haut eines Patienten aufgesetzt wird, und in Figur 16 in seinem zum Halten einer Hautwölbung 9 vorgesehenen Endzustand dargestellt. Dieses Ausführungsbeispiel weist als Mittel zum Erzeugen und Halten einer Hautwölbung 9 ebenfalls zwei gegeneinander bewegliche Halteteile 3a, 3b auf. Die Vorrichtung wird zunächst mit der Anlagefläche 7b des zweiten Halteteils 3b auf die Haut eines Patienten aufgesetzt. Die Anlagefläche 7b ist als eine Klebefläche ausgebildet, beispielsweise mittels einer Klebefolie 5. Anschließend wird das Halteteil 3a gegenüber dem Halteteil 3b verschwenkt, so dass das Halteteil mit seinem Andruckbereich 4 gegen die Haut des Patienten gedrückt wird. Dabei wird das Halteteil 3a noch oben bewegt bzw. das Halteteil 3a nach unten gedrückt, so dass zwischen der Anlagefläche 7b des Halteteils 3b und dem Andruckbereich 4 des Halteteils 3a ein Höhenunterschied und somit eine hangartig ansteigende Hautfläche entsteht.

In der in Figur 16 dargestellten Endposition deutet die Spitze einer Insertionsnadel durch eine Öffnung 11 in der Anlagefläche 7a des einen Halteteils 3a hindurch und in einem Abstand an der Anlagefläche 7b des anderen Halteteils 3b entlang. Zum Auslösen einer Stichs wird die Insertionsnadel also parallel zu der an der Anlagefläche 7b des zweiten Halteteils 3b anliegenden Hautoberfläche entlang in Unterhautfettgewebe eingeschoben.

Figur 17 zeigt ein weiteres Ausführungsbeispiel einer Insertionsvorrichtung 3, bei dem die Mittel zum Erhalten und Erzeugen einer Hautwölbung 9 von zwei gegeneinander beweglichen Halteteilen 3a, 3b der Insertionsvorrichtung 3 gebildet werden. Die beiden Halteteile sind bei dem in Figur 17 dargestellten Ausführungsbeispiel klammerartig gegeneinander beweglich und haben jeweils eine Auflagefläche 4, mit der die Halteteile gegen die Haut eines Patienten gedrückt werden. Die beiden Halteteile schwenken nach dem Aufsetzen auf die Haut eines Patienten aus einer Ausgangsstellung, bevorzugt unter Einwirkung der Rückstellkraft einer Feder, in einen Endzustand, in dem die beiden Auflageflächen 4 näher zueinander bewegt sind und deshalb die Haut zwischen sich aufgewölbt haben. Eine erzeugte Hautwölbung liegt dann an Anlageflächen 7a bzw. 7b der beiden Halteteile 3a, 3b an. Die Insertionsnadel 8 deutet in der Endposition in einem Abstand an den Anlageflächen 7a, 7b der beiden Halteteile 3a, 3bentlang, bevorzugt parallel an diesen entlang.

Figur 18 zeigt ein weiteres Ausführungsbeispiel einer Insertionsvorrichtung 3. Bei diesem Ausführungsbeispiel sind die Mittel zum Erhalten und Erzeugen einer Hautwölbung 9 als ein haubenartiges Gehäuse 12 ausgebildet, das mit einer umlaufenden Auflagefläche 4 gegen die Haut eines Patienten gedrückt wird und eine als Unterdruckanschluss ausgebildete Öffnung 13 aufweist, so dass eine Hautwölbung 9 in das haubenartige Gehäuse 12 hineingesaugt werden kann.

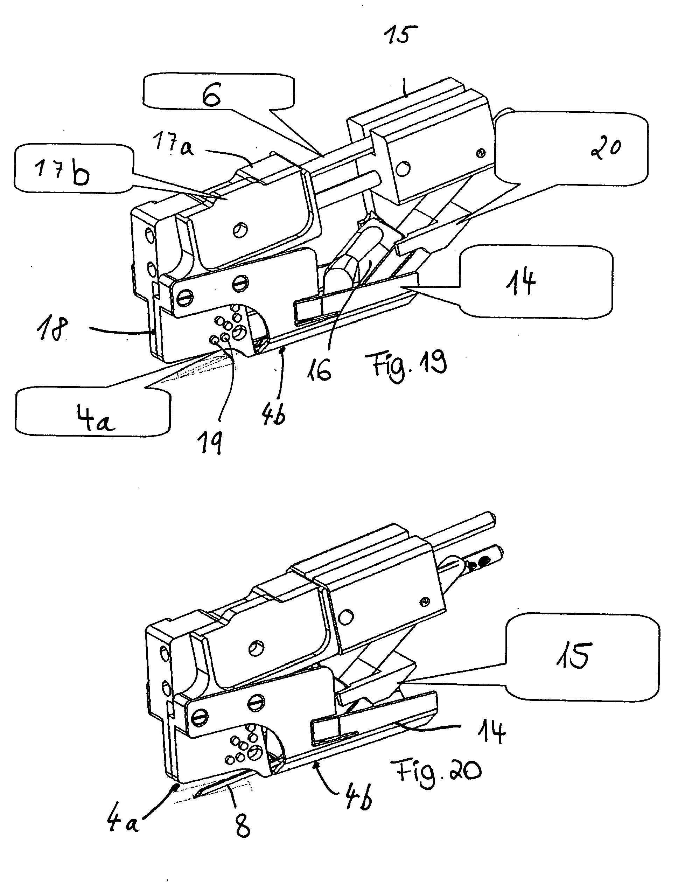

In Figur 19 ist ein weiteres Ausführungsbeispiel einer Insertionsvorrichtung dargestellt. Bei diesem Ausführungsbeispiel wird die Haut verformt, in dem die Unterseite der Insertionsvorrichtung gegen die Haut gedrückt wird. Die Unterseite weist einen ersten Andruckbereich 4a und einen zweiten Andruckbereich 4b auf, wobei sich zwischen den beiden Andruckbereichen 4a, 4b eine Aussparung, bevorzugt eine Öffnung, für die Insertionsnadel 8 befindet. Die beiden Andruckbereiche 4a, 4b sind ähnlich wie bei dem Ausführungsbeispiel der Figuren 6 und 7 auf unterschiedlichen Höhen angeordnet. Dies bedeutet, dass eine von oben nach unten verlaufende Schnittebene, in welcher die Insertionsnadel 8 liegt, den ersten Andruckbereich 4a in einer ersten Linie und den zweiten Andruckbereich 4b in einer zweiten Linie schneidet, und die erste Linie in der Nachbarschaft der Aussparung in einer größeren Höhe als die zweite Linie verläuft. Da die erwähnte Schnittebene die Nadel 8 enthält, verläuft die Schnittebene auch durch die Aussparung, so dass sich zwei durch die Aussparung getrennte Linien ergeben.

Die beiden Andruckbereiche 4a, 4b können kontinuierlich in einer rampenartig ansteigenden Fläche, in der die Aussparung für die Insertionsnadel 8 angeordnet ist, in einander übergehen oder scharf voneinander abgegrenzt sein.

Beim Andrücken der Unterseite der Insertionsvorrichtung gegen die Haut wird das Unterhautfettgewebe von den Andruckbereichen 4a, 4b komprimiert. Dabei wird das Gewebe von dem tiefer liegenden Andruckbereich 4b stärker komprimiert als von dem höher liegenden Andruckbereich, so dass die Haut zwischen den beiden Andruckbereichen eine hangartig ansteigende Fläche bildet, die an der Austrittsöffnung anliegt.

Der erste Andruckbereich 4a erstreckt sich bei dem dargestellten Ausführungsbeispiel weiter als sich die Insertionsnadel 8 in dem in Figur 20 dargestellten ausfahrenden Zustand, der bei einem Stich am Ende der Vorschubbewegung erreicht wird. Die Insertionsnadel 8 ist deshalb stets auf voller Länge von der Insertionsvorrichtung bedeckt.

Der erste Andruckbereich 4a ist ein geradliniger Streifen. Bevorzugt hat dieser Streifen eine Breite von weniger als 1 cm, insbesondere weniger als 6 mm. Auf diese Weise wird die Andruckkraft auf eine vorteilhaft kleine Fläche konzentriert, so dass sich mit geringem Kraftaufwand eine Gewebekomprimierung erreichen lässt. Der Andruckbereich 4b ist bei dem dargestellten Ausführungsbeispiel ebenfalls ein geradliniger Streifen. Die Insertionsvorrichtung wirkt beim Andrücken deshalb in einem streifenförmigen Bereich auf die Haut eines Patienten ein.

Der zweite Andruckbereich 4b ist dabei zwischen zwei Stützflächen 14 angeordnet, die sich sowohl seitlich weg von dem Andruckbereich 4b als auch nach oben erstrecken. Die Insertionsvorrichtung verbreitert sich also von ihrer Unterseite aus nach oben hin. Die seitlichen Stützflächen 14 wirken beim Andrücken der Insertionsvorrichtung als Abstandhalter für verdrängtes Gewebe, so dass ein größerer Arbeitsraum für eine Stechmechanik geschaffen wird.

Die Stechmechanik ist bei dem dargestellten Ausführungsbeispiel als ein Schlitten 15 ausgebildet, der bei einem Stich zusammen mit der von ihm gehaltenen Insertionsnadel 8 vorgeschoben wird. Der Schlitten 15 ist an einer Linearführung 6 geführt, die beispielsweise durch Führungsstangen gebildet sein kann. Der Schlitten 15 kann bei einem Stich einen Halter 20 des Sensorpatches 16 bewegen

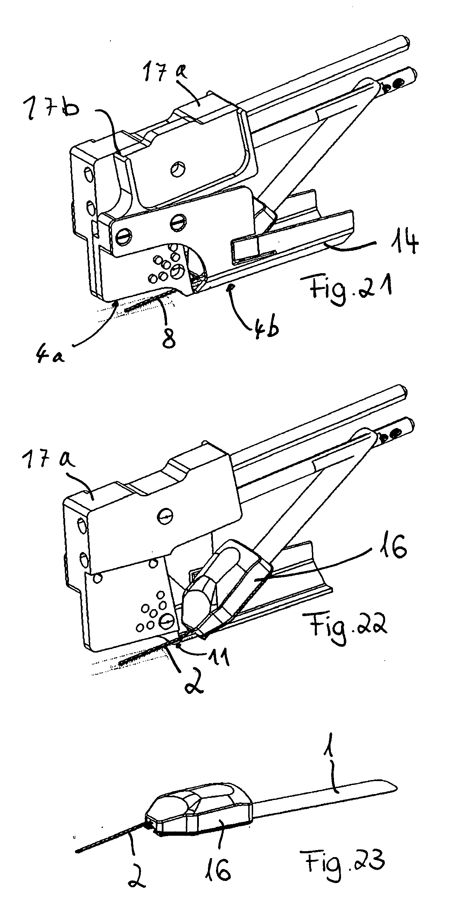

Nach einem Stich wird zunächst der Schlitten 15 von der Insertionsvorrichtung abgenommen. Figur 21 zeigt die Insertionsvorrichtung nach Abnehmen des Schlittens 15.

Die Insertionsvorrichtung ist aus zwei Teilkörpern 17a, 17b zusammengefügt. Zwischen dem ersten Teilkörper 17a und dem zweiten Teilkörper 17b verläuft eine Fuge 18 durch die Öffnung hindurch. Die Fuge 18 verläuft in Längsrichtung der Vorrichtung, also durch die beiden Andruckbereiche 4a, 4b. Im Prinzip kann sich die Fuge 18 aber auch quer durch die Insertionsvorrichtung erstrecken.

Die beiden Teilkörper 17a, 17b sind gegeneinander beweglich, damit die Insertionsvorrichtung nach dem Einstich der Insertionsnadel 8 leichter vom Körper eines Patienten abgenommen werden kann. Bei dem dargestellten Ausführungsbeispiel sind der erste und der zweite Teilkörper 17a, 17b durch Magnetkraft aneinander gehalten. Die Magnetkraft lässt sich manuell überwinden, so dass die beiden Teilkörper 17a, 17b bei Bedarf voneinander abgenommen werden können. Figur 22 zeigt den ersten Teilkörper 17a der Vorrichtung nach Abnehmen des zweiten Teilkörpers 17b. In einem weiteren Schritt wird auch der erste Teilkörper 17a entfernt. Und danach das Sensorpatch 16 auf der Haut aufgeklebt.

Um die beiden Teilkörper 17a, 17b durch Magnetkraft miteinander zu verbinden, können beiden Teilkörper 17a, 17b einen oder mehrere Permanentmagnete 19 tragen.

An sich genügt es jedoch, einen der beiden Teilkörper mit einem Permanentmagneten auszurüsten. Der andere Teilkörper kann statt eines Permanentmagneten auch einen Weichmagneten tragen, beispielsweise indem er an der betreffenden Stelle aus einem ferromagnetischen Stahl besteht.

Um das manuelle Überwinden der Magnetkraft zum Trennen der beiden Teilkörper 17a, 17b zu erleichtern, kann einer der beiden Teilkörper 17b an der Oberseite etwas über den anderen Teilkörper 17a hinausragen. Auf diese Weise kann eine Angriffstelle gebildet werden, beispielsweise um mit einem Daumen die zum Überwinden der magnetischen Anziehung erforderliche Kraft aufzubringen. Möglich ist es auch, dass sich die Fuge 18 zwischen den beiden Teilkörpern 17a, 17b an einer Stelle oder an mehreren Stellen nach oben hin keil oder trichterförmig verbreitert. Auf diese Weise kann mit einem Finger in die Fuge eingegriffen werden, um die beiden Teilkörper 17a, 17b auseinander zu drücken.

### Bezugszahlen

- 1: Hautoberfläche
- 2: Sensor
- 3: Insertionsvorrichtung
- 3a: Halteteil
- 3b: Halteteil
- 3c: Halteteil
- 4: Andruckbereich
- 5: Klebefolie
- 6: Linearführung
- 7a: Anlagefläche
- 7b: Anlagefläche
- 8: Insertionsnadel
- 9: Hautwölbung
- 10: Folienscharnier
- 11: Aussparung
- 12: Gehäuse
- 13: Unterdruckanschluss
- 15: Schlitten
- 16: Sensorpatch
- 17a: Teilkörper
- 17b: Teilkörper
- 18: Fuge
- 19: Magnet
- 20: Halter

## Patentansprüche

1. Verfahren zum Vorbereiten eines Einstichs einer Insertionsnadel (8) in Unterhautfettgewebe eines Patienten, wobei eine Insertionsvorrichtung (3), welche die Insertionsnadel (8) enthält, an die Haut eines Patienten angelegt wird, **dadurch gekennzeichnet, dass** die Haut durch Einwirkung der Insertionsvorrichtung (3) verformt und dabei eine Hautfläche (1a) stufen- oder hangartig ansteigend so angeordnet wird, dass die Insertionsnadel (8) mit ihrer Spitze auf die stufen- oder hangartig ansteigende Hautfläche (1a) deutet.

2. Verfahren nach Anspruch 1, **dadurch gekennzeichnet, dass** die Haut verformt wird, indem eine Unterseite der Insertionsvorrichtung gegen die Haut gedrückt wird,
wobei die Unterseite einen ersten Andruckbereich (4a) und einen zweiten Andruckbereich (4b) zum Andrücken an die Haut aufweist, und
sich zwischen dem ersten Andruckbereich (4a) und dem zweiten Andruckbereich (4b) eine Aussparung (11) für die Insertionsnadel (8) befindet, und
wobei eine von oben nach unten verlaufende Schnittebene, in welcher die Insertionsnadel (8) liegt, den ersten Andruckbereich (4a) in einer ersten Linie und den zweiten Andruckbereich (4b) in einer zweiten Linie schneidet, und
die erste Linie in der Nachbarschaft der Aussparung (11) in einer größeren Höhe als die zweite Linie verläuft.

3. Verfahren nach einem der vorstehenden Ansprüche, **dadurch gekennzeichnet, dass** die Hautoberfläche durch Einwirkung der Insertionsvorrichtung mindestens so stark verformt wird, dass eine Flächennormale in einem Punkt der ansteigenden Fläche, auf den die Insertionsnadel (8) deutet, mit einer Flächennormalen in demselben Punkt der Hautfläche vor Verformung einen Winkel von wenigstens 45°, vorzugsweise wenigstens 60°, einschließt.

4. Insertionsvorrichtung mit einer Unterseite zum Anlegen an die Haut eines Patienten, die eine Aussparung (11) für eine von der Insertionsvorrichtung gehaltene Insertionsnadel (8) aufweist, **gekennzeichnet durch** Mittel zum Formen einer stufen- oder hangartig ansteigen Hautfläche (1a), die der Aussparung (11) zugewandt ist, vorzugsweise an der Aussparung anliegt.

5. Insertionsvorrichtung nach Anspruch 4, **dadurch gekennzeichnet, dass** die Mittel zum Formen der stufen- oder hangartig ansteigenden Hautfläche (1a) einen ersten Andruckbereich (4a) und einen zweiten Andruckbereich (4b) zum Andrücken an die Haut umfassen, wobei sich zwischen dem ersten Andruckbereich (4a) und dem zweiten Andruckbereich (4b) die Aussparung (11) für die Insertionsnadel (8) befindet und wobei eine von oben nach unten verlaufende Schnittebene, in welcher die Insertionsnadel (8) liegt, den ersten Andruckbereich (4a) in einer ersten Linie und den zweiten Andruckbereich (4b) in einer zweiten Linie schneidet, und wobei die erste Linie in der Nachbarschaft der Aussparung (11) in einer größeren Höhe als die zweite Linie verläuft.

6. Insertionsvorrichtung nach Anspruch 5, **dadurch gekennzeichnet, dass** wenigstens einer der beiden Andruckbereiche (4a, 4b) ein Streifen ist.

7. Insertionsvorrichtung nach Anspruch 5 oder 6, **dadurch gekennzeichnet, dass** die Insertionsnadel (8) in einem ausgefahren Zustand, der bei einem Stich am Ende einer Vorschubbewegung erreicht wird, von dem ersten Andruckbereich (4a) bedeckt ist.

8. Insertionsvorrichtung nach einem der Ansprüche 4 bis 7, **dadurch gekennzeichnet, dass** sich die Insertionsvorrichtung von ihrer Unterseite aus nach oben hin verbreitert.

9. Insertionsvorrichtung nach Anspruch 8, **dadurch gekennzeichnet, dass** der zweite Andruckbereich (4b) zwischen zwei Stützflächen (14) angeordnet ist, die sich sowohl seitlich weg von dem zweiten Andruckbereich (4b) als auch nach oben erstrecken.

10. Insertionsvorrichtung nach einem der Ansprüche 4 bis 9, **dadurch gekennzeichnet, dass** die Mittel zum Formen der stufen- oder hangartigen Hautfläche eine Klebefläche (5) zum Aufkleben auf die zu formende Haut umfassen.

11. Insertionsvorrichtung nach einem der Ansprüche 4 bis 10, **dadurch gekennzeichnet, dass** die Mittel zum Formen der stufen- oder hangartigen Hautfläche der Haut wenigstens zwei Halteteile (3a, 3b) umfassen, die zum Formen der Haut gegeneinander beweglich sind.

12. Insertionsvorrichtung nach einem der Ansprüche 4 bis 11, **dadurch gekennzeichnet, dass** die Insertionsvorrichtung aus einem ersten und einem zweiten Teilkörper (17a, 17b) zusammengesetzt ist, wobei der erste und der zweite Teilkörper gegeneinander beweglich sind und eine Fuge (18) zwischen dem ersten und dem zweiten Teilkörper durch die Aussparung (11) hindurch verlauft.

13. Insertionsvorrichtung nach Anspruch 12, **dadurch gekennzeichnet, dass** der erste und der zweite Teilkörper (17a, 17b) durch Magnetkraft aneinander gehalten sind.

14. Insertionsvorrichtung nach Anspruch 12 oder 13 **dadurch gekennzeichnet, dass** die Fuge (18) in Längsrichtung der Insertionsvorrichtung verläuft.

15. Insertionsvorrichtung nach einem der Ansprüche 4 bis 14, **dadurch gekennzeichnet, dass** die Insertionsnadel (8) in der Insertionsvorrichtung schräg von oben nach unten gehalten ist.
